(19) Europäisches Patentamt
European Patent Office
Office européen des brevets

(11) **EP 3 958 776 B1**

(12) **EUROPEAN PATENT SPECIFICATION**

(45) Date of publication and mention
of the grant of the patent:
**11.09.2024   Bulletin 2024/37**

(21) Application number: **20722965.9**

(22) Date of filing: **15.04.2020**

(51) International Patent Classification (IPC):
***A61B 18/18*** (2006.01)

(52) Cooperative Patent Classification (CPC):
**A61B 18/1815;** A61B 2018/00023;
A61B 2018/00577; A61B 2018/1853;
A61B 2018/1892

(86) International application number:
**PCT/US2020/028188**

(87) International publication number:
**WO 2020/219307 (29.10.2020 Gazette 2020/44)**

(54) **MICROWAVE ABLATION DEVICE AND SYSTEM WITH IMPEDANCE MISMATCH**

MIKROWELLENABLATIONSVORRICHTUNG UND SYSTEM MIT IMPEDANZFEHLANPASSUNG

DISPOSITIF ET SYSTÈME D'ABLATION PAR MICRO-ONDES AVEC DÉSADAPTATION D'IMPÉDANCE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **26.04.2019   US 201962839008 P**

(43) Date of publication of application:
**02.03.2022   Bulletin 2022/09**

(73) Proprietor: **Covidien LP
Mansfield, MA 02048 (US)**

(72) Inventors:
• **DICKHANS, William J.
Longmont, Colorado 80503 (US)**
• **HALBUR, John J.
Woodridge, Illinois 60517 (US)**

(74) Representative: **Maschio & Soames IP Ltd
30 Carlton Crescent
Southampton SO15 2EW (GB)**

(56) References cited:
**WO-A1-2016/054156      GB-A- 2 559 604
US-A1- 2011 077 632      US-A1- 2014 290 830**

**Description**

CROSS-REFERENCE TO RELATED APPLICATIONS

This application claims the benefit of the filing date of provisional U.S. Patent Application No. 62/839,008, filed April 26, 2019.

BACKGROUND

Practitioners use conventional microwave ablation systems during laparoscopic surgical procedures to treat target tissue, such as a tumor located within the abdomen or pelvis, by using a percutaneous microwave ablation device to deliver microwave energy to the target tissue. An energy source, such as a generator, is coupled to the microwave ablation device via a generator cable. The microwave ablation device typically includes an elongated tubular member that contains a coaxial cable and that has a needle at its distal portion. The tubular member serves as a conduit for the coaxial cable, which guides microwave energy from the generator through an incision in the patient and toward the distal portion, which radiates the microwave energy toward the target tissue. To minimize reflected power and thereby optimize microwave energy delivery from the generator to the distal portion, conventional microwave ablation systems typically include a generator output port, a generator cable, and a microwave ablation device coaxial cable that have respective impedances that are mutually matched to one another within an operational frequency range. In this context, reference is made to document WO2016/054156 A1.

Recently, to reduce invasiveness and thereby reduce post-surgery healing time and the risk of bleeding or injury, practitioners have expressed a desire for a microwave ablation device having a tubular member that is reduced in outer diameter. One approach to reducing the diameter of the tubular member involves reducing respective outer diameters of a center conductor and a dielectric layer of the coaxial cable housed within the tubular member, to maintain the impedance of the coaxial cable despite its downsizing. This approach, however, results in excess loss in the coaxial cable and thereby decreases the efficiency of microwave energy delivery from the generator to the distal portion. In view of the foregoing, a need exists for a microwave ablation system including a microwave ablation device that has a tubular member that is reduced in diameter and that facilitates efficient delivery of microwave energy from the generator to the distal portion.

SUMMARY

The invention is defined in the appended set of claims. In one aspect, this disclosure describes a microwave ablation system comprising a generator, a generator cable, and a microwave ablation device. The generator comprises a generator output port having a generator output port impedance. The generator cable has a generator cable impedance that is matched to the generator output port impedance within a degree of tolerance within a predetermined frequency range. The microwave ablation device comprises a coaxial cable that has a coaxial cable impedance and is coupled to the generator output port by way of the generator cable. Within the predetermined frequency range, the coaxial cable impedance is lower than at least one of the generator output port impedance or the generator cable impedance by at least a predetermined amount that exceeds the degree of tolerance.

In embodiments, the microwave ablation device further comprises a transition waveguide and an electrically conductive element. The transition waveguide comprises a transition waveguide input port, having a transition waveguide input port impedance, and a transition waveguide output port, having a transition waveguide output port impedance. The coaxial cable comprises a coaxial cable input port and a coaxial cable output port, and the coaxial cable input port is coupled to the transition waveguide output port, and the coaxial cable output port is coupled to the electrically conductive element.

In embodiments, at least one of the transition waveguide input port impedance or the transition waveguide output port impedance is matched to the generator output port impedance and the generator cable impedance within the degree of tolerance within the predetermined frequency range.

In embodiments, at least one of the transition waveguide input port impedance or the transition waveguide output port impedance is matched to the coaxial cable impedance within the degree of tolerance within the predetermined frequency range.

In embodiments, the transition waveguide input port impedance, the transition waveguide output port impedance, the generator output port impedance, and the generator cable impedance are in a range from approximately 48 ohms ($\Omega$) to 52 $\Omega$, and the coaxial cable impedance is in a range from approximately 39 S2 to 45 $\Omega$.

In embodiments, the coaxial cable comprises a center conductor and a dielectric layer that radially surrounds the center conductor, and an outer diameter of the center conductor is in a range from approximately 0.0070 inches to 0.0072 inches and an outer diameter of the dielectric layer is in a range from approximately 0.0189 inches to 0.0195 inches.

In embodiments, the degree of tolerance is approximately 5 %.

In embodiments, the transition waveguide output port has a transition waveguide output port impedance that is matched to at least one of the transition waveguide input port impedance or the generator output port impedance within the degree of tolerance.

In embodiments, the microwave ablation system further comprises a balun comprising a balun insulator and a tubing member. The balun insulator has an inner diameter, and the tubing member radially surrounds the balun insulator and has a maximum outer diameter. The balun insulator inner diameter is approximately 0.023 inches, and the tubing member maximum outer diameter is approximately 0.041 inches.

In embodiments, the microwave ablation device further comprises an outer tubular member that houses the coaxial cable, and an outer diameter of the outer tubular member is approximately 15 gauge.

In embodiments, the predetermined frequency range includes at least one of 915 MHz, 2.45 GHz, or 5.8 GHz.

In another aspect, this disclosure describes a microwave ablation device comprising a transition waveguide, an electrically conductive element, and a coaxial cable. The transition waveguide comprises a transition waveguide input port, having a transition waveguide input port impedance, and a transition waveguide output port, having a transition waveguide output port impedance. The coaxial cable has a coaxial cable impedance and comprises a coaxial cable input port and a coaxial cable output port. The coaxial cable input port is coupled to the transition waveguide output port, and the coaxial cable output port is coupled to the electrically conductive element. Within a predetermined frequency range, the coaxial cable impedance is lower than at least one of the transition waveguide input port impedance or the transition waveguide output port impedance by at least a predetermined amount that exceeds a degree of tolerance.

In embodiments, the transition waveguide input port impedance and the transition waveguide output port impedance are in a range from approximately 48 S2 to 52 Ω, and the coaxial cable impedance is in a range from approximately 39 S2 to 45 Ω.

In embodiments, the coaxial cable comprises a center conductor and a dielectric layer that radially surrounds the center conductor, and an outer diameter of the center conductor is in a range from approximately 0.00070 inches to 0.00072 inches and an outer diameter of the dielectric layer is in a range from approximately 0.0189 inches to 0.0195 inches.

In embodiments, the microwave ablation device further comprises a balun comprising a balun insulator and a tubing member. The balun insulator has an inner diameter, and the tubing member radially surrounds the balun insulator and has a maximum outer diameter. The balun insulator inner diameter is approximately 0.023 inches, and the tubing member maximum outer diameter is approximately 0.041 inches.

In embodiments, the microwave ablation device further comprises an outer tubular member that houses the coaxial cable, and an outer diameter of the outer tubular member is in a range from approximately 0.0715 inches to 0.0725 inches.

BRIEF DESCRIPTION OF THE DRAWINGS

Various aspects and features of the present microwave ablation devices and systems are described herein below with references to the drawings, wherein:

FIG. 1 is a schematic diagram of a microwave ablation system, according to an embodiment of the present disclosure;

FIG. 2 shows an enlarged, cross-sectional view of the microwave ablation device shown in FIG. 1 in accordance with an embodiment of the present disclosure;

FIGS. 3A, 3B, and 3C show respective views of various portions of the microwave ablation device of FIGS. 1 and 2, and illustrate exemplary dimensions of components of the microwave ablation device, according to an embodiment of the present disclosure;

FIG. 4 is an enlarged, cross-sectional view of the indicated area of detail of FIG. 2, in accordance with an embodiment of the present disclosure;

FIG. 5A is an enlarged, cross-sectional view of a portion of a coaxial cable of a microwave ablation device at a first stage during the assembly process, in accordance with an embodiment of the present disclosure;

FIG. 5B is an enlarged, cross-sectional view of the portion of the coaxial cable shown in FIG. 5A, at a second stage of the assembly process, in accordance with an embodiment of the present disclosure;

FIG. 5C is an enlarged, cross-sectional view of the portion of the coaxial cable shown in FIG. 5A and 5B, at a third stage of the assembly process, in accordance with an embodiment of the present disclosure; and

FIG. 6 is a cross-sectional view of a portion of a probe assembly of the microwave ablation device, in accordance with an embodiment of the present disclosure.

DETAILED DESCRIPTION

The present disclosure is directed to a microwave ablation system including a microwave ablation device that has a tubular member that is reduced in outer diameter relative to tubular members of prior microwave ablation devices, and nonetheless facilitates efficient delivery, from the generator to the distal portion, of microwave energy suitable for effective treatment. The microwave ablation system and device of the present disclosure thus reduces invasiveness of microwave ablation procedures and thereby reduces post-surgery healing time and the risk of bleeding or injury. In general, as described in further detail below, the microwave ablation device of the present disclosure includes a coaxial cable (sometimes referred to as a feedline) that has an impedance lower than an impedance of one or more other components of the system, such as, for instance, the generator output port.

In one aspect, the impedance of the coaxial cable is reduced relative to the impedance of the generator output port by decreasing a ratio between an outer diameter of a dielectric layer of the coaxial cable to the outer diameter of the coaxial cable center conductor. In one embodiment, the decreased ratio, relative to a corresponding ratio for a coaxial cable that has an impedance that matches the generator output port impedance, is obtained by decreasing the outer diameter of the dielectric layer while keeping the center conductor outer diameter the same or greater. In another embodiment, the decreased ratio, relative to a corresponding ratio for a coaxial cable that has an impedance that matches the generator output port impedance, is obtained by increasing the center conductor outer diameter while keeping the outer diameter of the dielectric layer the same or lower. Despite the mismatched impedance between the coaxial cable of the present disclosure and the generator output port, the coaxial cable of the present disclosure includes a relatively large center conductor outer diameter, which has an increased thermal mass for absorbing heat and increased surface area for removal of the heat, and has a relatively thin insulating layer (e.g., PTFE), decreasing the amount that the insulating layer mitigates heat removal. The coaxial cable of the present disclosure thus provides performance advantages while minimizing the increase in heat buildup on the center conductor and reduces the risk of overheating the center conductor to the point of sudden and total failure (burnout). The relatively large center conductor also may cause less heat buildup on the center conductor and thereby reduce a risk of overheating the center conductor to the point of sudden and total failure or burnout.

Throughout this description, the term "proximal" refers to the portion of the device or component thereof that is closer to the clinician and the term "distal" refers to the portion of the device or component thereof that is farther from the clinician. The phrases "in an embodiment," "in embodiments," "in some embodiments," or "in other embodiments" may each refer to one or more of the same or different embodiments in accordance with the present disclosure. A phrase in the form "A or B" means "(A), (B), or (A and B)." A phrase in the form "at least one of A, B, or C" means "(A); (B); (C); (A and B); (A and C); (B and C); or (A, B, and C)."

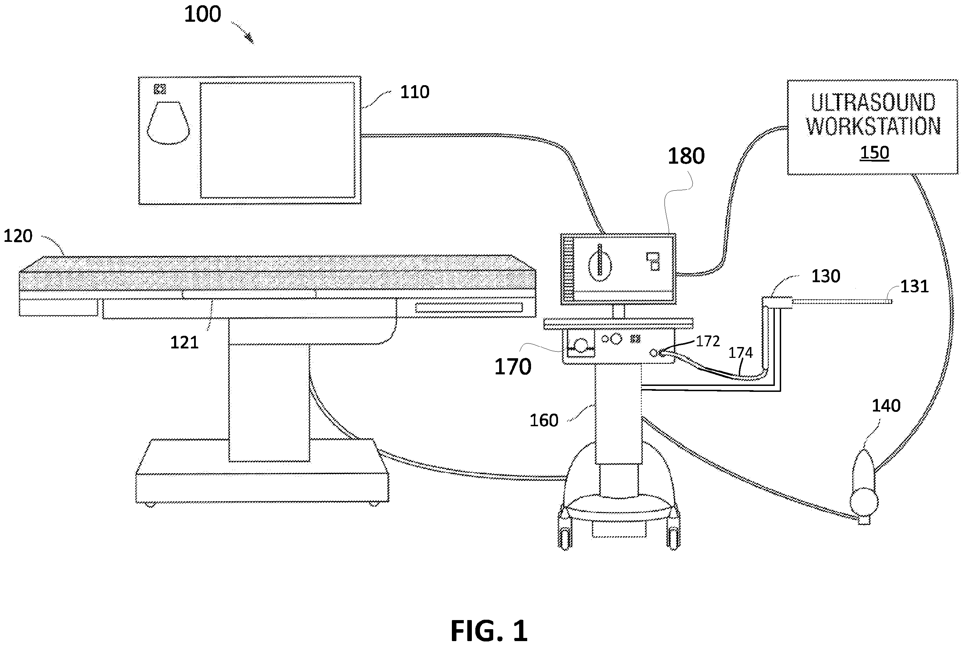

With reference to FIG. 1, a microwave ablation system 100 includes a display 110, a table 120 including an electromagnetic (EM) field generator 121, a microwave ablation device 130 including an EM sensor 131, an ultrasound sensor 140 connected to an ultrasound workstation 150, a peristaltic pump 160, and a computing device 180 attached to or in operable communication with a microwave generator 170. The computing device 180 may be, for example, a laptop computer, desktop computer, tablet computer, or other similar device. The computing device 180 may be configured to control the microwave generator 170, the peristaltic pump 160, a power supply (not shown in FIG. 1), and/or any other accessories and peripheral devices relating to, or forming part of, the microwave ablation system 100. The display 110 is configured to output instructions, images, and messages relating to the performance of the treatment procedure.

The table 120 may be, for example, an operating table or other table that is suitable for use during a treatment procedure and that includes the EM field generator 121. The EM field generator 121 is used to generate an EM field during the treatment procedure and forms part of an EM tracking system that is used to track positions of surgical instruments within the body of a patient, such as by tracking a position of the EM sensor 131. The EM field generator 121 may include various components, such as a specially designed pad to be placed under, or integrated into, an operating table or patient bed. An example of such an EM tracking system is the AURORA™ system sold by Northern Digital, Inc.

The microwave ablation device 130 is a surgical instrument for percutaneously accessing and treating a target location. The microwave ablation device 130 may include, or have attached to it, EM sensor 131 enabling the EM tracking system to track the location, position, and orientation (also known as the "pose") of the microwave ablation device 130 inside the body of the patient.

The microwave generator 170 includes a generator output port 172 and a generator cable 174 couples the generator output port 172 to the microwave ablation device 130. The microwave generator 170 delivers microwave energy from to the microwave ablation device 130 by way of the generator output port 172 and the generator cable 174. The generator output port 172 has a corresponding generator output port impedance and the generator cable 174 has a corresponding generator cable impedance. In some embodiments, to minimize reflected power, the impedance of the generator cable 174 is matched, within a degree of tolerance, to the impedance of the generator output port 172 within a predetermined frequency range.

In general, the microwave ablation system 100 is designed to operate using signals within a predetermined frequency range (sometimes referred to as an operational frequency range). That is, various components of the microwave ablation system 100, such as the microwave generator 170, microwave generator output port 172, generator cable 174, and microwave ablation device 130, are designed to generate, convey, radiate, or otherwise process signals (e.g., microwave signals) having a signal frequency that lies within the predetermined frequency range. The predetermined frequency range may be, for example, sufficiently wide to include a variety of operating frequencies, such as approximately 915 MHz, 2.45 GHz, and/or 5.8 GHz, or may be any other suitable frequency range.

Due to inconsistencies and/or imperfections in manufacturing, materials, variations in physical properties of components over signal frequency, and/or other factors, the various impedances of the microwave ablation system 100 and its components, such as the respective impedances of the generator output port 172 and the generator cable 174, may vary from their nominal impedance values throughout the operational frequency range. The degree of tolerance, in some embodiments, is predetermined in recognition of this fact. The degree of tolerance, as used herein, generally refers to a permissible limit of variation in impedances that would not significantly affect the function of the microwave ablation system 100. The degree of tolerance, for example, may be expressed as a variation in impedance amount relative to a nominal impedance value (such as a maximum number of $\Omega$2 greater than or less than the nominal impedance value) or as a variation in impedance percentage relative to the nominal impedance value (such as, a maximum percentage greater than or less than the nominal impedance value). In various embodiments, the degree of tolerance may be, for example, approximately $\pm$ 3 $\Omega$, $\pm$ 5 %, or any other suitable value.

In addition to outputting microwave energy, the microwave generator 170 is configured to control the peristaltic pump 160, which is configured to pump fluid through the microwave ablation device 130, cooling the microwave ablation device 130 during operation. While the present disclosure describes the use of the microwave ablation system 100 in a surgical environment, it is also envisioned that some or all of the components of the microwave ablation system 100 may be used in alternative settings, for example, an imaging laboratory and/or an office setting.

In addition to the EM tracking system, the surgical instruments may also be visualized by using ultrasound imaging. The ultrasound sensor 140, such as an ultrasound wand, may be used to image the patient's body during the treatment procedure to visualize the location of the surgical instruments, such as the microwave ablation device 130, inside the patient's body. The ultrasound sensor 140 may have an EM tracking sensor embedded within or attached to the ultrasound wand, for example, a clip-on sensor or a sticker sensor. As described further below, the ultrasound sensor 140 may be positioned in relation to the microwave ablation device 130 such that the microwave ablation device 130 is at an angle to the ultrasound image plane, thereby enabling the clinician to visualize the spatial relationship of the microwave ablation device 130 with the ultrasound image plane and with objects being imaged. Further, the EM tracking system may also track the location of the ultrasound sensor 140. In some embodiments, one or more of the ultrasound sensors 140 may be placed inside the body of the patient. The EM tracking system may then track the location of such ultrasound sensors 140 and the microwave ablation device 130 inside the body of the patient. The ultrasound workstation 150 may be used to configure, operate, and view images captured by the ultrasound sensor 140. Likewise, the computing device 180 may be used to configure, operate, and view images captured by the ultrasound sensor 140, either directly or relayed via the ultrasound workstation 150.

In embodiments, the microwave ablation device 130 is used to ablate a lesion or tumor (hereinafter referred to as a "target") by using microwave energy to heat tissue in order to denature or kill cancerous cells. The construction and use of a system including such an ablation probe is more fully described in U.S. Patent Publication No. 2016/0058507, entitled MICROWAVE ABLATION SYSTEM, filed on August 26, 2014, by Dickhans; U.S. Patent No. 9,247,992, entitled MICROWAVE ABLATION CATHETER AND METHOD OF UTILIZING THE SAME, filed on March 15, 2013, by Ladtkow et al.; and U.S. Patent No. 9,119,650, entitled MICROWAVE ENERGY-DELIVERY DEVICE AND SYSTEM, filed on March 15, 2013, by Brannan et al.

As noted above, the location of the microwave ablation device 130 within the body of the patient may be tracked during the treatment procedure. An example method of tracking the location of the microwave ablation device 130 is by using the EM tracking system, which tracks the location of the microwave ablation device 130 by tracking sensors, such as the EM sensor 131, attached to or incorporated in the microwave ablation device 130. Various types of sensors may be used, such as a printed sensor, the construction and use of which is more fully described in U.S. Patent Publication No. US 2016/0174873, entitled "MEDICAL INSTRUMENT WITH SENSOR FOR USE IN A SYSTEM AND METHOD FOR ELECTROMAGNETIC NAVIGATION", filed on October 22, 2015, by Greenburg et al. A percutaneous treatment system similar to the above-described microwave ablation system 100 is further described in U.S. Patent Application Publication No. 2016/0317224, entitled "MICROWAVE ABLATION PLANNING AND PROCEDURE SYSTEMS", filed on April 15, 2016, by Girotto et al.

While the above-described microwave ablation system 100 uses a microwave generator 170 to provide microwave energy to the microwave ablation device 130, those skilled in the art will appreciate that various other types of generators and tools may be used without departing from the scope of the present disclosure. For example, radio frequency (RF) ablation tools receiving RF energy from RF generators may be substituted for the microwave generators and ablation tools described above. Further, while the above-described microwave ablation system 100 is designed for percutaneous access to tissue, those skilled in the art will appreciate that the methods described below may be used with systems and tools designed for endobronchial navigation to access treatment locations via the patient's airways and surrounding parenchyma without departing from the scope of the present disclosure. An example of such an endobronchial navigation system is described in U.S. Patent Application Publication No. 2016/0000302, entitled "SYSTEM AND METHOD FOR NAVIGATING WITHIN THE LUNG", filed on June 29, 2015, by Brown et al.

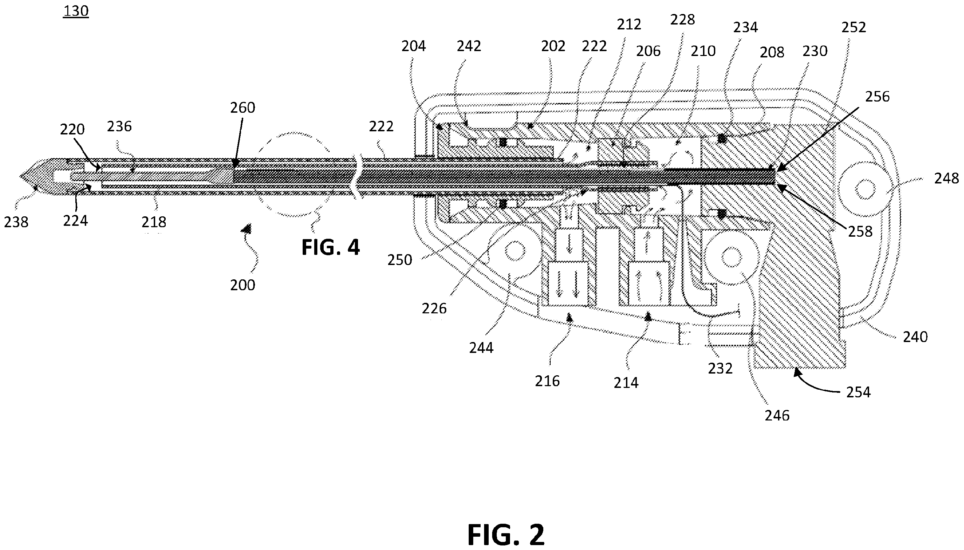

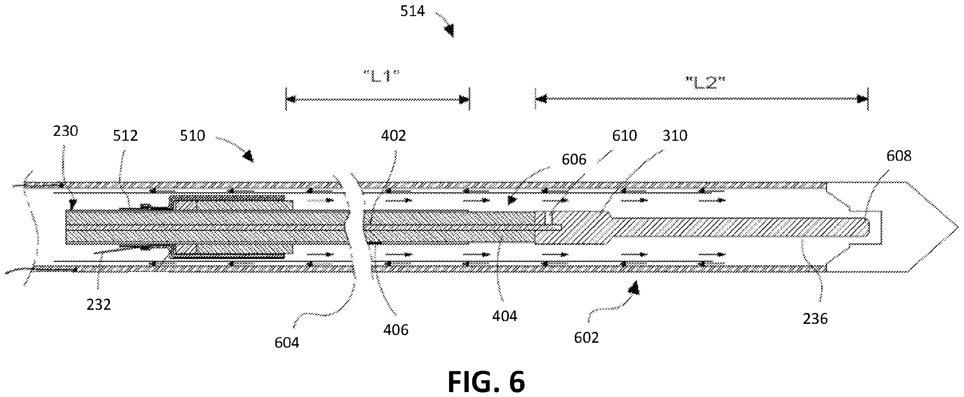

FIG. 2 shows a side cross-sectional view of a portion of an example embodiment of the microwave ablation device 130 shown in FIG. 1. Additional aspects of the microwave ablation device 130 shown in FIG. 2 are described in U.S. Patent No. 9,119,650, entitled "MICROWAVE ENERGY DELIVERY DEVICE AND SYSTEM," filed on March 15, 2013, by Brannan et al. The microwave ablation device 130 includes an outer tubular member 222, an inner tubular member 218, a coaxial cable 230, an antenna assembly (shown in FIG. 6), and a tip 238, which, when assembled, form a probe assembly 200, or portions thereof. The microwave ablation device 130 generally includes two housing halves (not separately shown in FIG. 2), which, when assembled, form a handle body 240. The handle body 240 defines a handle-body chamber (not separately shown in FIG. 2) therein. The microwave ablation device 130 includes a hub 202 (as well as other components described herein) disposed, at least in part, within the handle-body chamber.

The hub 202 includes a hub body 208 defining a hub-body chamber (not separately shown in FIG. 2) therein. The microwave ablation device 130 includes a hub cap 204 and a hub divider 206, which are configured to be receivable within the hub-body chamber in sealing engagement with the inner walls of the hub body. The outer tubular member 222, the inner tubular member 218, the hub 202, and the components cooperative therewith (e.g., hub cap 204 and hub divider 206) are adapted to maintain fluid flow toward the electrically-conductive element 236. The hub body 208 generally includes a first port 214 and a second port 216 to allow fluid communication with a coolant supply system (for example, the peristaltic pump 160 shown in FIG. 1) via one or more coolant paths. The first port 214 and the second port 216 may be of any suitable shape, such as rectangular, cylindrical, and/or the like, and may include a groove adapted to receive an O-ring or other suitable sealing element.

In some embodiments, the hub body 208 may include one or more mechanical interfaces, such as a recess 242, adapted to matingly engage with one or more corresponding mechanical interfaces associated with the handle body 240 to align the hub 202 within the handle body 240 and/or to fixedly secure the hub 202 within the handle-body chamber. Similarly, each of the housing halves may include a series of mechanical interfacing components, such as alignment pins 244, 246, and 248, configured to matingly engage with a corresponding series of mechanical interfaces (not shown in FIG. 2) to align the two housing halves about the components and assemblies of the microwave ablation device 130.

The hub divider 206 is configured and utilized to divide the hub-body chamber into a first chamber 210 disposed in fluid communication with the first port 214, and a second chamber 212 disposed in fluid communication with the second port 216. The first chamber 210 generally fluidly connects the first port 214 to the inner tubular member 218. The second chamber 212 generally fluidly connects the second port 216 to the inner tubular member 222.

In some embodiments, the inner walls of the hub body 208 may include a configuration of engagement portions adapted to provide sealing engagement with the hub cap 204 and/or the hub divider 206. An O-ring 250 may be provided for engagement with the hub cap 204. The O-ring 250 may provide sealing force that permits flexing and/or other slight movement of the hub cap 204 relative to the hub 202 under fluid-pressure conditions.

The outer tubular member 222 and the inner tubular member 218 may be formed of any suitable non-electrically-conductive material, such as, for example, polymeric or ceramic materials. In some embodiments, as shown in FIG. 2, the inner tubular member 218 is coaxially disposed around the coaxial cable 230 and defines a first lumen 220 therebetween, and the outer tubular member 222 is coaxially disposed around the inner tubular member 218 and defines a second lumen 224 therebetween.

The probe assembly 200 generally includes an antenna assembly 600 (shown in FIG. 6 but not separately shown in FIG. 2) having a first radiating portion (e.g., distal radiating section 602 shown in FIG. 6) and a second radiating portion (e.g., proximal radiating section 604 shown in FIG. 6). The antenna assembly 600, which is described in more detail below, is operably coupled by the coaxial cable 230 to a transition waveguide 252 shown in FIG. 2, which is adapted to transmit microwave energy from the cable assembly 174 to the coaxial cable 230. The transition waveguide 252 has a transition waveguide input port 254 having a transition waveguide input port impedance, and a transition waveguide output port 256 having a transition waveguide output port impedance. The coaxial cable 230 has a coaxial cable impedance and includes a coaxial cable input port 258, and a coaxial cable output port 260. The coaxial cable input port 258 is coupled to the transition waveguide output port 256, and the coaxial cable output port 260 is coupled to the electrically-conductive element 236.

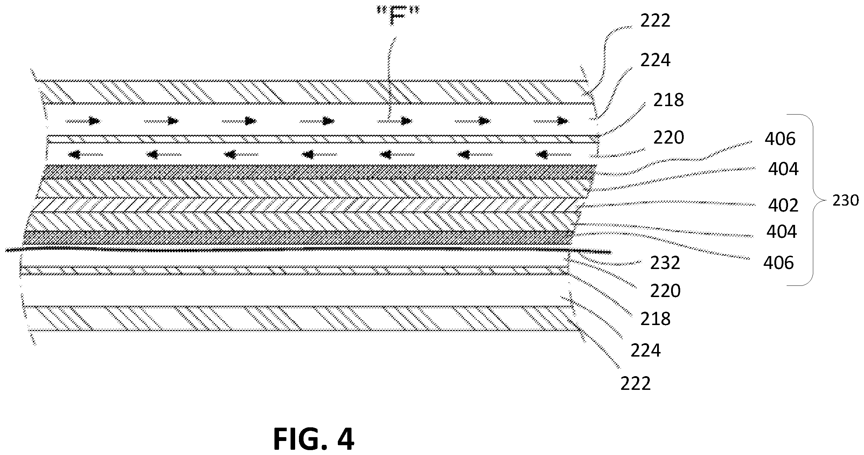

The coaxial cable 230 may be any suitable transmission line, e.g., a coaxial cable. In some embodiments, as shown in FIG. 4, the coaxial cable 230 includes an inner conductor 402, an outer conductor 406 coaxially disposed around the inner conductor 402, and a dielectric material 404 disposed therebetween. The dielectric material 404 may be formed from any suitable dielectric material, e.g., polyethylene, polyethylene terephthalate, polyimide, or polytetrafluoroethylene (PTFE). The inner conductor 402 and the outer conductor 406 may be formed from any suitable electrically-conductive material. In some embodiments, the inner conductor 402 is formed from a first electrically-conductive material (e.g., stainless steel) and the outer conductor 406 is formed from a second electrically-conductive material (e.g., copper). Electrically-conductive materials used to form the coaxial cable 230 may be plated with other materials, e.g., other conductive materials, such as gold or silver, to improve their properties, e.g., to improve conductivity, decrease energy loss. The coaxial cable 230 may have any suitable length defined between its proximal and distal ends. In accordance with various embodiments of the present disclosure, the coaxial cable 230 is coupled at its proximal end to the transition waveguide 252 and coupled at its distal end to the antenna assembly 600. The coaxial cable 230 is disposed at least in part within the inner tubular member 218.

As shown in FIG. 2, the probe assembly 200 is disposed in part within the hub 202, wherein the hub cap 204 and the hub divider 206 are disposed in sealing engagement with the inner walls of the hub body, and a proximal portion of the probe assembly 200 is disposed in association with the hub cap 204 and hub divider 206. The hub divider 206 generally divides the hub-body chamber (not separately shown in FIG. 2) into the first chamber 210 and the second chamber 212. The first chamber 210 is disposed in fluid communication with the first port 214. The second chamber 212 is disposed in fluid communication with the second port 216. In some embodiments, as shown in FIG. 2, the proximal end of the inner tubular member 218 is disposed within the first chamber 210, wherein the first lumen 220 is disposed in fluid communication with the first port 214, and the proximal end of the outer tubular member 222 is disposed within the second chamber 212, wherein the second lumen 224 is disposed in fluid communication with the second port 216.

In some embodiments, as shown in FIGS. 2 and 4, the inner tubular member 218 is coaxially disposed around a coaxial cable 230 and defines the first lumen 220 therebetween, and the outer tubular member 222 is coaxially disposed around the inner tubular member 218 and defines the second lumen 224 therebetween. As shown in FIG. 2, the proximal end of the inner tubular member 218 is disposed within the first chamber 210, the first lumen 220 is disposed in fluid communication with the first port 214, the proximal end of the outer tubular member 222 is disposed within the second chamber 212, and the second lumen 224 is disposed in fluid communication with the second port 216.

In some embodiments, as shown in FIG. 2, the inner tubular member 218 includes a first portion having a first outer diameter, a second portion having a second outer diameter greater than the first outer diameter, and a neck portion 226 disposed therebetween. In some embodiments, the opening in the hub divider 206 is configured for sealing engagement with the second portion of the inner tubular member 218 having the second outer diameter. In some embodiments, located within the interior of the second portion of the inner tubular member 218 is a high hoop strength metal cylinder 228. The metal cylinder 228 engages the inner diameter of the inner tubular member 218. The hub divider 206 is formed of an elastomeric material and when forced into place within the hub 202, as shown in FIG. 2, the elastomeric material of the hub divider 206 creates an improved water tight seal separating the first hub chamber 210 from the second hub chamber 212. The metal cylinder 228 improves this seal by ensuring better contact between the elastomeric material of the hub divider 206 and the inner tubular member 218 upon application of lateral forces to the hub divider 206.

The hub body 208 may be configured to sealingly engage the coolant supply lines forming coolant paths to fluid inlet port 214 and fluid outlet port 216. The fluid inlet port 214 and the fluid outlet port 216 may have any suitable configuration, including without limitation nipple-type inlet fittings, compression fittings, and recesses, and may include an O-ring type elastomeric seal.

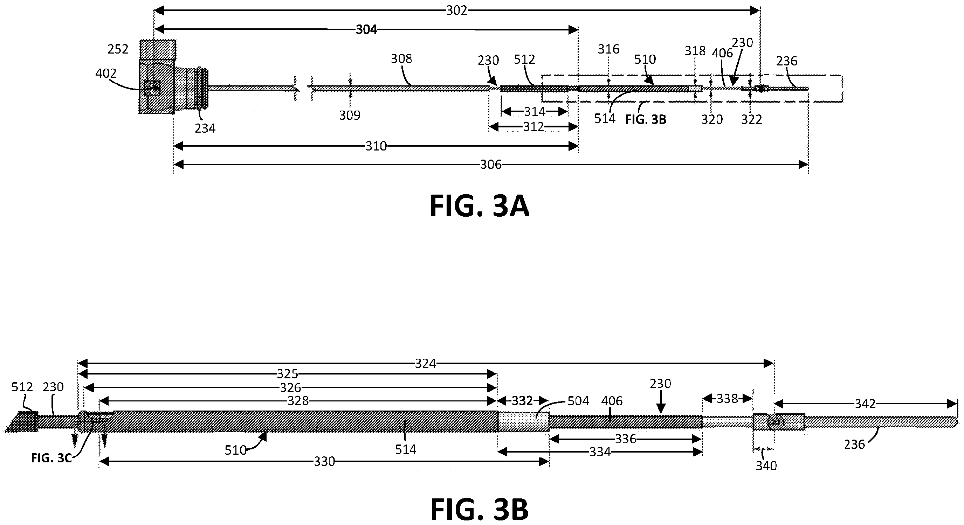

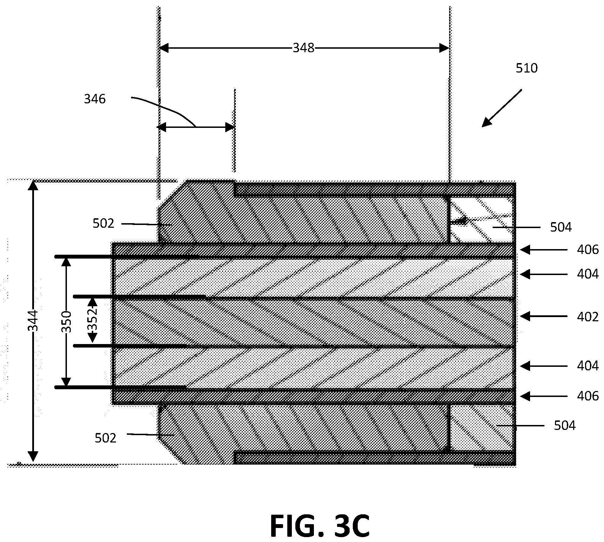

FIGS. 3A, 3B, and 3C, which show respective views of various portions of the microwave ablation device 130 of FIGS. 1 and 2, illustrate exemplary dimensions of components of the microwave ablation device 130, in accordance with embodiments of the present disclosure. In particular, FIG. 3A shows an assembly including the coaxial cable 230 having the transition waveguide 252 coupled to its proximal end, having the electrically conductive element 236 coupled to its distal end, and having a balun 510 disposed adjacent to, and on a proximal side of, the electrically-conductive element 236. FIG. 3B shows an enlarged view of a distal portion of the assembly shown in FIG. 3A. FIG. 3C shows an enlarged cross-sectional view of a proximal portion of the balun 510 identified in FIG. 3B.

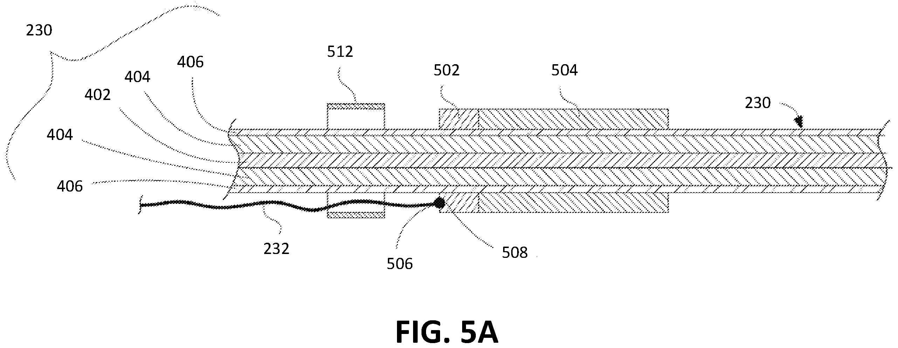

As shown in FIG. 3A, a tubular member 308, which in some embodiments may be a stainless steel tube, is coaxially disposed around the coaxial cable 230 and is partially disposed within the transition waveguide 252. A tubing member 512, which, in some embodiments is a heat-shrink tubing member (described below in connection with FIGS. 5A-5C) is coaxially disposed around the coaxial cable 230 in a position that is distal to that of the tubular member 308. The balun 510 is coaxially disposed around the coaxial cable 230 in a position that is distal to that of the heat-shrink tubing member 512. And the electrically conductive element 236 is coupled to the coaxial cable 230 at its distal end.

In general, various dimensions of the microwave ablation device 130 may vary in various embodiments. The view in FIG. 3A illustrates various exemplary dimensions of the microwave ablation device 130 and corresponding degrees of tolerance for those dimensions. In particular, a dimension 302 from a proximal end portion of the center conductor 402 to a distal end portion of the center conductor 402 may be, for example, approximately 8.8 inches in one embodiment or approximately 6.833 inches in another embodiment. A dimension 304 from the proximal end portion of the center conductor 402 to a distal end portion of the balun 510 may be, for example, approximately 7.438 inches in one embodiment or approximately 5.471 inches in another embodiment. A dimension 306 from a proximal portion of the transition waveguide 252 to a distal end portion of the electrically-conductive element 236 may be, for example, approximately 9.016 inches in one embodiment or approximately 7.049 inches in another embodiment. An outer diameter 309 of the tubular member 308 may be, for example, approximately 0.0343 inches. An outer diameter 309 of the tubular member 308 may be, for example, approximately 0.0343 inches. A dimension 310 from the proximal portion of the transition waveguide 252 to a distal end portion of the balun 510 may be, for example, approximately 7.294 inches in one embodiment or approximately 5.327 inches in another embodiment. A dimension 312 from a distal end portion of the tubular member 308 to the proximal end portion of the balun 510 may be, for example, approximately 0.670 inches. A dimension 314 from a distal end portion of the heat-shrink tubing member 512 to a distal end portion of the heat-shrink tubing member 512 may be, for example, approximately 0.50 inches. An outer diameter 316 of a middle portion of the balun 510 may be, for example, approximately 0.041 inches. An outer diameter 318 of a distal portion of the balun 510, more particularly, of a dielectric layer 504 (described below in connection with FIGS. 5A-5C) of the balun 510 exposed at its distal portion, may be, for example, approximately 0.0375 inches. An outer diameter 320 of the outer conductor 406 of the coaxial cable 230 may be, for example, approximately 0.0230 inches. An outer diameter 322 of exposed dielectric material 404 of the coaxial cable 230 may be, for example, approximately 0.0192 inches.

Referring now to FIG. 3B, a dimension 324 from the proximal end portion of the balun 510 to a distal end portion of the center conductor 402 may be, for example, approximately 1.362 inches. A dimension 325 from the proximal end portion of the balun 510 to a distal end portion of a tubing member 514 of the balun 510 may be, for example, approximately 0.822 inches. In various embodiments, the tubing member 514 may be a heat-shrink tubing member, a metal (e.g., copper) tubing member crimped in place, and/or the like. A dimension 326 from the proximal end portion of the tubing member 514 of the balun 510 to the distal end portion of the tubing member 514 of the balun 510 may be, for example, approximately 0.811 inches. A dimension 328 from a proximal end portion of the dielectric layer 504 of the balun 510 to the distal end portion of the tubing member 514 of the balun 510 may be, for example, approximately 0.780 inches. A dimension 330 from a proximal end portion of the dielectric layer 504 of the balun 510 to a distal end portion of the dielectric layer 504 of the balun 510 may be, for example, approximately 0.880 inches. A dimension 332 from a proximal end of an exposed portion of the dielectric layer 504 to a distal end portion of the exposed portion of the dielectric layer 504 may be, for example, approximately 0.100 inches. A dimension 334 from the proximal end of the exposed portion of the dielectric layer 504 to a distal end portion of the outer conductor 406 may be, for example, approximately 0.400 inches. A dimension 336 from a distal end of the exposed portion of the dielectric layer 504 to the distal end portion of the outer conductor 406 may be, for example, approximately 0.300 inches. A dimension 338 from the distal end portion of the outer conductor 406 to a distal end portion of the exposed dielectric material 404 of the coaxial cable 230 may be, for example, approximately 0.100 inches. A dimension 340 from the distal end portion of the exposed dielectric material 404 of the coaxial cable 230 to the distal end portion of the center conductor 402 may be, for example, approximately 0.040 inches. A dimension 342 from the distal end portion of the center conductor 402 to the distal end portion of the electrically-conductive element 236 may be, for example, approximately 0.040 inches.

Referring now to FIG. 3C, an outer diameter 344 of the balun at its proximal end portion may be, for example, approximately 0.0410 inches. A dimension 346 from a proximal end portion of the balun short 502 to the proximal end portion of the tubing member 514 may be, for example, approximately 0.011 inches. A dimension 348 from the proximal end portion of the balun short 502 to a distal end portion of balun short 502 may be, for example, approximately 0.042 inches.

As mentioned above, in some embodiments, the microwave ablation device 130 has an outer tubular member 222 (not shown in FIGS. 3A-3B) that is reduced in outer diameter relative to outer tubular members of prior microwave ablation devices. In some examples, for instance, the outer tubular member 222 is approximately 15 gauge in diameter, or has an outer diameter in a range from approximately 0.074 inches +/-0.0035 inches or from approximately 0.074 inches +/-0.002 inches. The microwave ablation system 100 and device 130 thus reduces invasiveness of microwave ablation procedures and thereby reduces post-surgery healing time and the risk of bleeding or injury. Despite the reduction in size of the outer tubular member 222, however, the microwave ablation system 100 and microwave ablation device 130 facilitate the efficient delivery of microwave energy from the generator 170 to the antenna assembly 600 for effective treatment. In particular, the microwave ablation system 100 and device 130 are designed such that the coaxial cable 230 includes a relatively large outer diameter 350 of the center conductor 402, even though maintaining a relatively large outer diameter 350 of the center conductor 402 while downsizing the outer tubular member 222, in at least some instances, gives rise to an impedance mismatch along a signal path from the generator 170 to the coaxial cable 230. Nonetheless, even with an impedance mismatch located at a particular portion along the signal path from the generator 170 to the coaxial cable 230, the microwave ablation system 100 and device 130 of the present disclosure yields performance advantages while minimizing the increase in energy loss to the antenna assembly 600 relative to that that would result by using a coaxial cable that has a impedance that is matched to the impedance of the generator output port 172 but that has a reduced outer diameter of its center conductor, in part because the coaxial cable 230 of the present disclosure maintains a relatively large outer diameter 350 of the center conductor 402.

As noted above, the impedance mismatch may be located between the coaxial cable 230 and one or more other components of the microwave ablation system 100. For example, the impedance mismatch may be located (1) at a junction where the coaxial cable 230 is coupled to the transition waveguide 252, (2) at a portion internal to the transition waveguide 252, or (3) at a junction where the transition waveguide 252 is coupled to the generator cable 174. In this manner, the impedance of the coaxial cable 230 may be mismatched with (for example, by being at least a predetermined amount lower than) at least one of the impedances of the generator 170, the generator output port 172, the transition waveguide input port 254, and/or the transition waveguide output port 256.

In some embodiments, for example, the impedance mismatch is located at a junction where the coaxial cable 230 is coupled to the transition waveguide 252, or, more particularly, where the input port 258 of the coaxial cable 230 is coupled to the output port 256 of the transition waveguide 252. For instance, the respective impedances of the transition waveguide output port 256, the transition waveguide input port 254, the generator cable 174, and the generator output port 172 may be matched to one another within the degree of tolerance and within the predetermined frequency range, and the impedance of the coaxial cable 230 may be lower than the respective impedances of the transition waveguide output port 256, the transition waveguide input port 254, the generator cable 174, and the generator output port 172 by at least a predetermined amount, which exceeds the degree of tolerance. In particular, in one embodiment, the impedance of the coaxial cable 230 is in a range from approximately 39 $\Omega$ to 45 $\Omega$, and the respective impedances of the transition waveguide output port 256, the transition waveguide input port 254, the generator output port 172, and the generator cable 174 are in a range from approximately 48 S2 to 52 $\Omega$, although each of these ranges may be smaller or larger in other embodiments.

In another embodiment, the impedance mismatch is located at a portion of the transition waveguide 252 between the transition waveguide input port 254 and the transition waveguide output port 256. For instance, within the predetermined frequency range, the impedance of the coaxial cable 230 may be matched to the impedance of the transition waveguide output port 256, and the respective impedances of the transition waveguide input port 254, the generator cable 174, and the generator output port 172 may be matched to one another within the degree of tolerance. In particular, in one illustrative embodiment, the respective impedances of the coaxial cable 230 and the transition waveguide output port 256 may be in a range from approximately 39 $\Omega$ to 45 $\Omega$, and the respective impedances of the transition waveguide input port 254, the generator cable 174, and the generator output port 172 may be in a range from approximately 48 S2 to 52 $\Omega$.

In yet another embodiment, the impedance mismatch is located at a junction where the transition waveguide 252 is coupled to the generator cable 174, or, more particularly, where the transition waveguide input port 254 is coupled to the generator cable 174. For instance, within the predetermined frequency range, the impedance of the coaxial cable 230 may be matched to the respective impedances of the transition waveguide output port 256 and the transition waveguide input port 254, and the respective impedances of the generator cable 174 and the generator output port 172 may be matched to one another within the degree of tolerance. In particular, in one embodiment, the respective impedances of the coaxial cable 230, the transition waveguide output port 256, and the transition waveguide input port 254 may be in a range from approximately 39 $\Omega$ to 45 $\Omega$, and the respective impedances of the generator cable 174 and the generator output port 172 may be in a range from approximately 48 S2 to 52 $\Omega$.

Referring now to the coaxial cable 230, which may be solid conductor or a hollow conductor, the approximate characteristic impedance $Z_0$ of the coaxial cable 230 can be computed according to equation (1), shown below, where $OD_{center\ conductor}$ represents the outer diameter 352 of the center conductor 402 of the coaxial cable 230, $OD_{dielectric}$ represents the outer diameter 350 of the dielectric layer 404 of the coaxial cable 230, and $\varepsilon_r$ represents the dielectric constant of the dielectric layer 404.

$$(1) \qquad Z_0(\Omega) \approx \frac{138}{\sqrt{\varepsilon_r}} \log_{10} \frac{OD_{dielectric}}{OD_{center\ conductor}}$$

In some embodiments, the impedance of the coaxial cable 230 is reduced relative to one or more other components of the microwave ablation system 100 by decreasing the ratio of the outer diameter 350 of the dielectric layer 404 to the outer diameter of the center conductor 402 of the coaxial cable 230. For example, the decreased ratio (relative to a corresponding ratio for a coaxial cable that has an impedance that matches the impedance of the generator output port 172) is obtained by decreasing the outer diameter 350 of the dielectric layer 404 while (1) keeping the outer diameter 352 of the center conductor 402 constant or (2) increasing the outer diameter 352 of the center conductor 402. In another embodiment, the decreased ratio is obtained by increasing the outer diameter 352 of the center conductor 402 while (1) keeping the outer diameter 350 of the dielectric layer 404 constant or (2) decreasing the outer diameter 350 of the dielectric layer 404. In some embodiments, the outer diameter 352 of the center conductor 402 is in a range from approximately 0.00070 inches to 0.00072 inches, the outer diameter 350 of the dielectric layer 404 is in a range from approximately 0.0189 inches to 0.0195 inches, and the dielectric constant $\varepsilon_r$ of the dielectric layer 404 is 2.01, thereby causing the coaxial cable 230 to have an impedance in a range from approximately 39 $\Omega$ to 45 $\Omega$.

Referring now to FIG. 4, in some embodiments, the first lumen 220 is utilized as a fluid inflow conduit and the second lumen 224 is utilized as a fluid outflow conduit. In other embodiments, the first lumen 220 may serve as a fluid outflow conduit and the second lumen 224 may serve as a fluid inflow conduit. The outer tubular member 222 and/or the inner tubular member 218 may be adapted to circulate coolant fluid therethrough, and may include baffles, multiple lumens, flow restricting devices, or other structures that may redirect, concentrate, or disperse flow depending on their shape. The size and shape of the inner tubular member 218, the outer tubular member 222, the first lumen 220, and the second lumen 224 may be varied from the configuration depicted in FIGS. 2 and 4.

FIG. 4 shows a portion of the probe assembly 200 of the microwave ablation device 130 of FIG. 2 including the first lumen 220, shown disposed between the outer tubular member 222 and the inner tubular member 218, the second lumen 224, shown disposed between the inner tubular member 218 and the coaxial cable 230, and a transmission line 232 extending longitudinally within the second lumen 224. The coaxial cable 230 is a coaxial cable that includes a center conductor 402. As indicated by the direction of the arrow-headed lines in FIG. 4, the first lumen 220 serves as an inflow conduit for coolant fluid "F" and the second lumen 224 serves as an outflow conduit for coolant fluid "F," however as noted above these could be reversed without departing from the scope of the present disclosure. In general, the impedance of the coaxial cable 230 may be determined by the inner diameter of the outer conductor 406, the outer diameter of the center conductor 402, and the dielectric constant of the dielectric material 404, and reducing the inner diameter of the outer conductor 406 causes the impedance of the coaxial cable 230 to decrease.

FIG. 5A shows a portion of the coaxial cable 230 including the inner conductor 402, the outer conductor 406 coaxially disposed around the inner conductor 402, and the dielectric material 404 disposed therebetween, shown with a balun short 502 coaxially disposed around a portion of the outer conductor 406. During assembly, the balun short 502 is coupled, deposited or otherwise formed onto, or joined to, the outer conductor 406. The balun short 502 may be formed as a single structure and electrically coupled to the outer conductor 406, for example, by solder or other suitable electrical connection. The balun short 502 may be formed of any suitable electrically-conductive materials, such as copper, gold, silver, or other conductive metals or metal alloys. In some embodiments, the balun short 502 has a generally ring-like or truncated tubular shape. The balun short 502 is electrically coupled to the outer conductor 406 of the coaxial cable 230 by any suitable manner of electrical connection, e.g., soldering, welding, or laser welding. The size and shape of the balun short 502 may be varied from the configuration depicted in FIG. 5A.

FIG. 5A further depicts a dielectric layer 504 (also referred to herein as a balun insulator) coaxially disposed around the outer conductor 406 and coupled thereto. The balun insulator 504 may be formed of any suitable insulative material, including, but not limited to, ceramics, water, mica, polyethylene, polyethylene terephthalate, polyimide, polytetrafluoroethylene (PTFE) (e.g., Teflon®, manufactured by E. I. du Pont de Nemours and Company of Wilmington, Del., United States), glass, metal oxides or other suitable insulator, and may be formed in any suitable manner. In some embodiments, as shown in FIG. 5A, the balun insulator 504 is a dielectric sleeve. The balun insulator 504 may be grown, deposited or formed by any other suitable technique. In some embodiments, the balun insulator 504 is formed from a material with a dielectric constant in the range of about 1.7 to about 10.

FIG. 5A further depicts a temperature sensor 506 disposed in contact with a proximal end of the balun short 502. The temperature sensor 506 is coupled to the transmission line 232 extending generally along a longitudinal axis of the coaxial cable 230. In some embodiments, the temperature sensor 506 is a thermocouple and the transmission line 232 is a thermocouple wire. The thermocouple wire may be a two lead wire thermocouple wire, for example it may be comprised of an insulated (anodized) side-by-side constantine wire and a copper wire. The balun short 502 may include an engagement element 508 adapted to engage with the temperature sensor 506, for example, to facilitate electrical and mechanical coupling of the temperature sensor 506 and the balun short 502. In some embodiments, the engagement element 508 may be a groove, slot, or recess cut into the balun short 502. Alternatively, the temperature sensor 506 may be soldered to balun short 502. Placement of the thermocouple 506 directly against the balun short 502 improves the sensitivity and thermo-profiling characteristics of the microwave ablation device 130, particularly as compared to traditional thermocouples in microwave ablation devices, which measure the temperature of the cooling fluid. As will be appreciated by those of skill in the art, the temperature of the coolant will lag the temperature of the balun itself, and thus provide only approximate indications of the temperature of the elements which are heated during operation. As a result, in instances where little or no coolant is flowing, the temperature of the balun 510 and coaxial cable 230 associated therewith can increase faster than that of the coolant and result in damage to the microwave ablation device 130 even before triggering a shut-off of the microwave ablation system 100 based on the temperature of the coolant. Accordingly, improved safety and performance can be achieved by direct sensing of temperature of the balun 510.

Still further, FIG. 5A depicts a heat-shrink tubing member 512 disposed in a first configuration around the outer conductor 406. During assembly, the heat-shrink tubing member 512 is utilized to secure a portion of the transmission line 232 to the coaxial cable 230. The heat-shrink tubing member 512 may be any suitable tubing material with the capability to respond to heat and bind around an object, and may have any suitable length. In some embodiments, the heat-shrink tubing member 512 may be a thermoplastic.

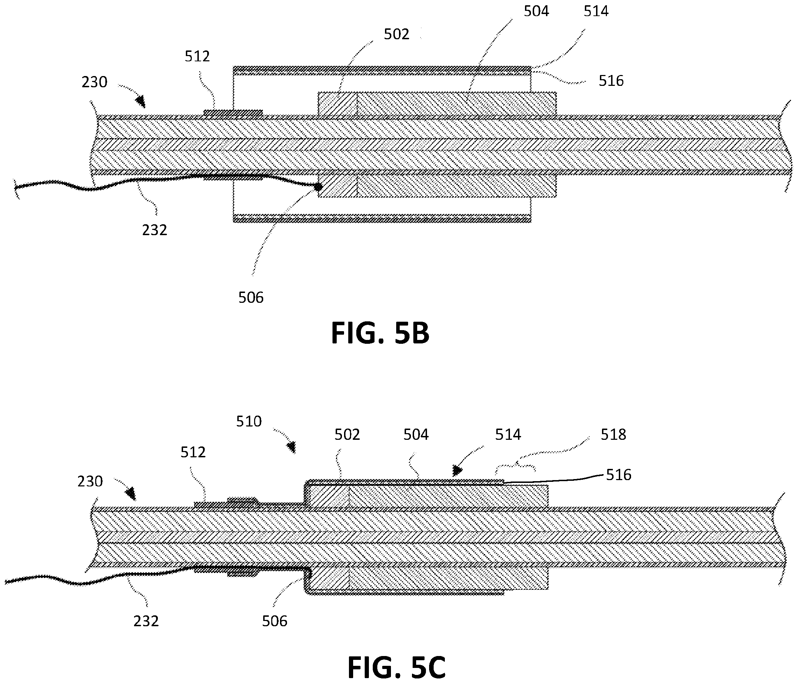

FIG. 5B shows the coaxial cable 230 of FIG. 5A following application of heat to the heat-shrink tubing member 512. During assembly, securing a portion of the transmission line 232 to the coaxial cable 230, as shown in FIG. 5C keeps the transmission line stable and helps to maintain the electrical and mechanical coupling of the temperature sensor 506 and the balun short 502 during subsequent assembly operations. FIG. 5B further shows a second tubing member 514 disposed in a first configuration.

The tubing member 514 includes an inner layer of an electrically-conductive material 516. The electrically-conductive layer 516 may be formed of any suitable electrically-conductive material, such as metallic material. In one embodiment the metallic material of electrically-conductive layer 516 is formed of a silver ink deposited or layered on an interior surface of the tubing member 514. The tubing member 514 may have a length from approximately 1 to 3 inches in length. However, the shape and size of the tubing member 514 and the balun insulator 504 may be varied from the configuration depicted in FIG. 5B without departing from the scope of the present disclosure. Indeed, though described as one embodiment, the orientation and implementation of the feed line 230 as well as other aspects of the present disclosure are not so limited. For example, the feed line 230 may incorporate one or more aspects of the ablation system described in U.S. Patent No. 9,247,992, filed on Mar. 15, 2013, entitled "MICROWAVE ABLATION CATHETER AND METHOD OF UTILIZING THE SAME".

FIG. 5C shows the balun 510 after the application of thermal energy or crimping, as the case may be, to the tubing member 514 and the resultant shrinkage or deformation. As shown FIG. 5C, the electrically-conductive material 516 is disposed in intimate contact with the balun short 502 and a portion of the balun insulator 504. In some embodiments, as shown in FIG. 5C, a portion of the balun insulator 504 may extend distally beyond the distal end of the tubing member 514 and electrically conductive layer 516, to create a gap 518. The gap 518 improves the microwave performance of the probe assembly 200 and can assist in achieving a desired ablation pattern. More specifically, the gap 518 ensures adequate coupling of microwave energy from a proximal radiating section 604 (FIG. 6) into the balun 510, improving the performance of the balun 510 over a wide range of tissue dielectric conditions. Further, FIG. 5C shows the tubing member 514 securing the portion of the transmission line 232 between the heat-shrink tubing member 512 and the balun short 502 to the coaxial cable 230 preventing its movement and substantially preventing the temperature sensor 506 from being removed from physical contact with the balun short 502.

In some embodiments, the balun insulator 504 has an inner diameter, and the tubing member 514, which radially surrounds the balun insulator 504, has a maximum outer diameter 344, which is its diameter at its largest portion. For example, the balun insulator 504 inner diameter may be approximately 0.041 inches, and the maximum outer diameter of the tubing member 514 may match the maximum outer diameter 344 of the tubing member 514 and may be approximately 0.041 inches. In this manner, despite having an outer diameter of the tubing member 514 that is relatively small compared to prior baluns of microwave ablation devices, the balun 510 is able to have a relatively large thickness, because the thickness of the outer diameter of the coaxial cable 230 has been decreased. This relatively thick size of the balun 510 facilitates efficient and concentrated delivery of energy to the antenna assembly 600 of the microwave ablation device 130, despite the downsizing of the microwave ablation device 130 relative to prior devices.

FIG. 6 is a cross-sectional view of a portion of the probe assembly 200 illustrating the balun 510 of FIG. 5C connected to the antenna assembly 600, in accordance with an embodiment of the present disclosure. In operation, microwave energy having a wavelength, lambda ($\lambda$), is transmitted through the antenna assembly 600 and radiated into the surrounding medium, e.g., tissue. The length of the antenna assembly 600 for efficient radiation may be dependent on the effective wavelength, $\lambda_{eff}$, which is dependent upon the dielectric properties of the treated medium. The antenna assembly 600 through which microwave energy is transmitted at a wavelength $\lambda$, may have differing effective wavelengths, $\lambda_{eff}$, depending upon the surrounding medium, e.g., liver tissue as opposed to breast tissue, lung tissue, kidney tissue, etc.

The antenna assembly 600, according to the embodiment shown in FIG. 6, includes a proximal radiating section 604 having a length "L1", a distal radiating section 602 including an electrically-conductive element 236 having a length "L2", and a feed point 606 disposed therebetween. In some embodiments, the proximal radiating section 604 may have a length "L1" in a range from approximately 0.05 inches to about 0.80 inches. The electrically-conductive element 236 may be formed of any suitable electrically-conductive material, e.g., metal such as stainless steel, aluminum, titanium, copper, or the like. In some embodiments, the electrically-conductive element 236 may have a length "L2" in a range from about 0.15 inches to about 1.0 inches.

As shown in FIG. 6, the electrically-conductive element 236 has a stepped configuration, such that the outer diameter of the distal portion 608 is less than the outer diameter of the proximal portion 310. Further, the inner conductor 402 of the coaxial cable 230 is arranged such that it extends past the distal end of the insulator 404 and into the proximal portion 310 of the electrically-conductive element 236. A hole 610 formed in the proximal portion 310 approximately at 90 degrees to the inner conductor 402 allows for solder, a set screw, or other securing mechanisms to physically secure the electrically-conductive element 236 to the inner conductor 402 and therewith the coaxial cable 230 of the microwave ablation device 130.

The embodiments disclosed herein are examples of the disclosure and may be embodied in various forms. For instance, although certain embodiments herein are described as separate embodiments, each of the embodiments herein may be combined with one or more of the other embodiments herein. Specific structural and functional details disclosed herein are not to be interpreted as limiting, but as a basis for the claims and as a representative basis for teaching one skilled in the art to variously employ the present disclosure in virtually any appropriately detailed structure. Like reference numerals may refer to similar or identical elements throughout the description of the figures.

The foregoing description is only illustrative of the present microwave ablation systems and devices. Various alternatives and modifications can be devised by those skilled in the art without departing from the disclosure. Accordingly, the present disclosure is intended to embrace all such alternatives, modifications and variances. The embodiments described with reference to the attached drawing figures are presented only to demonstrate certain examples of the disclosure. Other elements, steps, methods, and techniques that are insubstantially different from those described above and/or in the appended claims are also intended to be within the scope of the disclosure.

**Claims**

1. A microwave ablation system, comprising:

   a generator (170), comprising:
   a generator output port (172) having a generator output port impedance;
   a generator cable (174) having a generator cable impedance,
   wherein the generator cable impedance is matched to the generator output port impedance within a degree of tolerance within a predetermined frequency range; and
   a microwave ablation device (130), comprising:

   a coaxial cable (230) that has a coaxial cable impedance and is coupled to the generator output port by way of the generator cable,
   wherein, within the predetermined frequencyrange, the coaxial cable impedance is lower than at least one of the generator output port impedance or the generator cable impedance by at least a predetermined amount that exceeds the degree of tolerance,
   **characterized in that** the coaxial cable impedance is in a range from approximately 37 $\Omega$ to 47 $\Omega$.

2. The microwave ablation system of claim 1, wherein the microwave ablation device further comprises:

   a transition waveguide (252) comprising:

   a transition waveguide input port (254) having a transition waveguide input port impedance, and
   a transition waveguide output port (256) having a transition waveguide output port impedance; and

   an electrically conductive element (236),
   wherein the coaxial cable comprises

   a coaxial cable input port (258), and
   a coaxial cable output port (260),

   and wherein the coaxial cable input port is coupled to the transition waveguide output port, and the coaxial cable output port is coupled to the electrically conductive element.

3. The microwave ablation system of claim 2, wherein at least one of the transition waveguide input port impedance or the transition waveguide output port impedance is matched to the generator output port impedance and the generator cable impedance within the degree of tolerance within the predetermined frequency range.

4. The microwave ablation system of claim 2, wherein at least one of the transition waveguide input port impedance or the transition waveguide output port impedance is matched to the coaxial cable impedance within the degree of tolerance within the predetermined frequency range.

5. The microwave ablation system of claim 2, wherein the transition waveguide input port impedance and the transition waveguide output port impedance are in a range from approximately 48 S2 to 52 $\Omega$.

6. The microwave ablation system of claim 1, wherein the generator output port impedance and the generator cable impedance are in a range from approximately 48 S2 to 52 S2.

7. The microwave ablation system of claim 1, wherein the coaxial cable comprises a center conductor and a dielectric layer that radially surrounds the center conductor, wherein an outer diameter of the center conductor is in a range from approximately 0.1778 mm (0.0070 inches) to 0.18288 mm (0.0072 inches) and an outer diameter of the dielectric layer is in a range from approximately 0.4801 mm (0.0189 inches) to 0.4953 (0.0195 inches).

8. The microwave ablation system of claim 1, wherein the degree of tolerance is approximately 5 %.

9. The microwave ablation system of claim 2, wherein the transition waveguide output port has a transition waveguide output port impedance that is matched to at least one of the transition waveguide input port impedance or the generator output port impedance within the degree of tolerance.

10. The microwave ablation system of claim 1, further comprising:

   a balun (510) comprising:

   a balun insulator (504) having an inner diameter; and
   a tubing member (514) that radially surrounds the balun insulator and that has a maximum outer diameter,

   wherein the balun insulator inner diameter is in a range from approximately 0.5715 mm (0.0225 inches) to 0.5969 mm (0.0235 inches), and
   the tubing member maximum outer diameter is in a range from approximately 1.00 mm (0.0395 inches) to 1.08 mm (0.0425 inches).

11. The microwave ablation system of claim 1, wherein the microwave ablation device further comprises an outer tubular member (222) that houses the coaxial cable, and an outer diameter of the outer tubular member is in a range from approximately 1.8288 mm (0.072 inches) to 1.9685 mm (0.0775 inches).

12. The microwave ablation system of claim 1, wherein the predetermined frequency range includes at least one of 915 MHz, 2.45 GHz, or 5.8 GHz.

**Patentansprüche**

1. Mikrowellenablationssystem, umfassend:
   einen Generator (170), umfassend:
   einen Generatorausgangsport (172) mit einer Generatorausgangsport-Impedanz:

   ein Generatorkabel (174) mit einer Generatorkabel-Impedanz,
   wobei die Generatorkabel-Impedanz innerhalb eines Toleranzgrades in einem vorbestimmten Frequenzbereich auf die Generatorausgangsport-Impedanz abgestimmt ist;
   und eine Mikrowellenablationsvorrichtung (130), umfassend:

   ein Koaxialkabel (230), das eine Koaxialkabel-Impedanz aufweist und über das Generatorkabel an den Generatorausgangsport gekoppelt ist,
   wobei, innerhalb des vorbestimmten Frequenzbereichs, die Koaxialkabel-Impedanz um wenigstens eine vorbestimmte Menge, die den Toleranzgrad überschreitet, geringer ist als wenigstens eines von der Generatorausgabeport-Impedanz oder der Generatorkabel-Impedanz, **dadurch gekennzeichnet, dass** die Koaxialkabel-Impedanz in einem Bereich von annähernd 37 Ω bis 47 Ω liegt.

2. Mikrowellenablationssystem nach Anspruch 1, wobei die Mikrowellenablationsvorrichtung ferner umfasst:
   einen Übergangswellenleiter (252), umfassend:

   einen Übergangswellenleiter-Eingangsport (254) mit einer Übergangswellenleiter-Eingangsport-Impedanz, und
   einen Übergangswellenleiter-Ausgangsport (256) mit einer Übergangswellenleiter-Ausgangsport-Impedanz, und
   ein elektrisch leitfähiges Element (236),

wobei das Koaxialkabel umfasst:

einen Koaxialkabel-Eingangsport (258) und
einen Koaxialkabel-Ausgangsport (260),
und wobei der Koaxialkabel-Eingangsport an den Übergangswellenleiter-Ausgangsport gekoppelt ist und der Koaxialkabel-Ausgangsport an das elektrisch leitfähige Element gekoppelt ist.

3. Mikrowellenablationssystem nach Anspruch 2, wobei wenigstens eines von der Übergangswellenleiter-Eingangsport-Impedanz oder der Übergangswellenleiter-Ausgangsport-Impedanz innerhalb des Toleranzgrades in dem vorbestimmten Frequenzbereich auf die Generatorausgabeport-Impedanz und die Generatorkabel-Impedanz abgestimmt ist.

4. Mikrowellenablationssystem nach Anspruch 2, wobei wenigstens eines von der Übergangswellenleiter-Eingangsport-Impedanz oder der Übergangswellenleiter-Ausgangsport-Impedanz innerhalb des Toleranzgrades in dem vorbestimmten Frequenzbereich auf die Koaxialkabel-Impedanz abgestimmt ist.

5. Mikrowellenablationssystem nach Anspruch 2, wobei die Übergangswellenleiter-Eingangsport-Impedanz und die Übergangswellenleiter-Ausgangsport-Impedanz in einem Bereich von annähernd 48 Ω bis 52 Ω liegen.

6. Mikrowellenablationssystem nach Anspruch 1, wobei die Generatorausgabeport-Impedanz und die Generatorkabel-Impedanz in einem Bereich von annähernd 48 Ω bis 52 Ω liegen.

7. Mikrowellenablationssystem nach Anspruch 1, wobei das Koaxialkabel einen Mittelleiter und eine dielektrische Schicht umfasst, die den Mittelleiter radial umgibt, wobei ein Außendurchmesser des Mittelleiters in einem Bereich von annähernd 0,1778 mm (0,0070 Zoll) bis 0,18288 mm (0,0072 Zoll) liegt und ein Außendurchmesser der dielektrischen Schicht in einem Bereich von annähernd 0,4801 mm (0,0189 Zoll) bis 0,4953 (0,0195 Zoll) liegt.

8. Mikrowellenablationssystem nach Anspruch 1, wobei der Toleranzgrad annähernd 5 % beträgt.

9. Mikrowellenablationssystem nach Anspruch 2, wobei der Übergangswellenleiter-Ausgangsport eine Übergangswellenleiter-Ausgangsport-Impedanz aufweist, die, innerhalb des Toleranzgrades, auf wenigstens eines von der Übergangswellenleiter-Eingangsport-Impedanz oder der Generatorausgabeport-Impedanz abgestimmt ist.

10. Mikrowellenablationssystem nach Anspruch, ferner umfassend:
ein Symmetrierglied (Balun) (510), umfassend:

einen Balun-Isolator (504) mit einem Innendurchmesser; und
ein Rohrleitungselement (514), das den Balun-Isolator radial umgibt und das einen maximalen Außendurchmesser aufweist,
wobei der Innendurchmesser des Balun-Isolators in einem Bereich von annähernd 0,5715 mm (0,0225 Zoll) bis 0,5969 mm (0,0235 Zoll) liegt
und
der maximale Außendurchmesser des Rohrleitungselements in einem Bereich von annähernd 1,00 mm (0,0395 Zoll) bis 1,08 mm (0,0425 Zoll) liegt.

11. Mikrowellenablationssystem nach Anspruch 1, wobei die Mikrowellenablationsvorrichtung ferner ein äußeres röhrenförmiges Element (222) umfasst, welches das Koaxialkabel beherbergt, und ein Außendurchmesser des äußeren röhrenförmigen Elements in einem Bereich von annähernd 1,8288 mm (0,072 Zoll) bis 1,9685 mm (0,0775 Zoll) liegt.

12. Mikrowellenablationssystem nach Anspruch 1, wobei der vorbestimmte Frequenzbereich wenigstens eines von 915 MHz, 2,45 GHz oder 5,8 GHz beinhaltet.

## Revendications

1. Système d'ablation par micro-ondes, comprenant :

un générateur (170), comprenant :

un port de sortie de générateur (172) ayant une impédance de port de sortie de générateur ;
un câble de générateur (174) ayant une impédance de câble de générateur,
dans lequel l'impédance de câble de générateur est adaptée à l'impédance de port de sortie de générateur avec un degré de tolérance dans une plage de fréquence prédéterminée ; et

un dispositif d'ablation par micro-ondes (130), comprenant :

un câble coaxial (230) qui a une impédance de câble coaxial et est couplé au port de sortie de générateur au moyen du câble de générateur,
dans lequel, dans la plage de fréquence prédéterminée, l'impédance de câble coaxial est inférieure à au moins l'une de l'impédance de port de sortie de générateur et de l'impédance de câble de générateur d'au moins une quantité prédéterminée qui dépasse le degré de tolérance,

**caractérisé en ce que** l'impédance de câble coaxial est dans une plage d'environ 37 Ω à 47 Ω.

2. Système d'ablation par micro-ondes selon la revendication 1, dans lequel le dispositif d'ablation par micro-ondes comprend en outre :
un guide d'ondes de transition (252) comprenant :

un port d'entrée de guide d'ondes de transition (254) ayant une impédance de port d'entrée de guide d'ondes de transition, et
un port de sortie de guide d'ondes de transition (256) ayant une impédance de port de sortie de guide d'ondes de transition ; et
un élément électroconducteur (236),
dans lequel le câble coaxial comprend

un port d'entrée de câble coaxial (258), et
un port de sortie de câble coaxial (260),

et dans lequel le port d'entrée de câble coaxial est couplé au port de sortie de guide d'ondes de transition, et le port de sortie de câble coaxial est couplé à l'élément électroconducteur.

3. Système d'ablation par micro-ondes selon la revendication 2, dans lequel au moins l'une de l'impédance de port d'entrée de guide d'ondes de transition et de l'impédance de port de sortie de guide d'ondes de transition est adaptée à l'impédance de port de sortie de générateur et à l'impédance de câble de générateur dans les limites du degré de tolérance dans la plage de fréquence prédéterminée.

4. Système d'ablation par micro-ondes selon la revendication 2, dans lequel au moins l'une de l'impédance de port d'entrée de guide d'ondes de transition et de l'impédance de port de sortie de guide d'ondes de transition est adaptée à l'impédance de câble coaxial dans les limites du degré de tolérance dans la plage de fréquence prédéterminée.

5. Système d'ablation par micro-ondes selon la revendication 2, dans lequel l'impédance de port d'entrée de guide d'ondes de transition et l'impédance de port de sortie de guide d'ondes de transition sont dans une plage d'environ 48 Q à 52 Q.

6. Système d'ablation par micro-ondes selon la revendication 1, dans lequel l'impédance de port de sortie de générateur et l'impédance de câble de générateur sont dans une plage d'environ 48 Q à 52 Q.

7. Système d'ablation par micro-ondes selon la revendication 1, dans lequel le câble coaxial comprend un conducteur central et une couche diélectrique qui entoure radialement le conducteur central, dans lequel un diamètre externe du conducteur central est dans une plage d'environ 0,1778 mm (0,0070 pouce) à 0,18288 mm (0,0072 pouce) et un diamètre externe de la couche diélectrique est dans une plage d'environ 0,4801 mm (0,0189 pouce) à 0,4953 (0,0195 pouce).

8. Système d'ablation par micro-ondes selon la revendication 1, dans lequel le degré de tolérance est d'environ 5 %.

9. Système d'ablation par micro-ondes selon la revendication 2, dans lequel le port de sortie de guide d'ondes de transition a une impédance de port de sortie de guide d'ondes de transition qui est adaptée à au moins l'une de l'impédance de port d'entrée de guide d'ondes de transition et de l'impédance de port de sortie de générateur dans les limites du degré de tolérance.

10. Système d'ablation par micro-ondes selon la revendication 1, comprenant en outre :

un symétriseur (510) comprenant :

un isolant de symétriseur (504) ayant un diamètre interne ; et
un élément tube (514) qui entoure radialement l'isolant de symétriseur et qui a un diamètre externe maximum,

dans lequel le diamètre interne de l'isolant de symétriseur est dans une plage d'environ 0,5715 mm (0,0225 pouce) à 0,5969 mm (0,0235 pouce), et
le diamètre externe maximal de l'élément tube est dans une plage d'environ 1,00 mm (0,0395 pouce) à 1,08 mm (0,0425 pouce).

11. Système d'ablation par micro-ondes selon la revendication 1, dans lequel le dispositif d'ablation par micro-ondes comprend en outre un élément tubulaire externe (222) qui abrite le câble coaxial, et un diamètre externe de l'élément tubulaire externe est dans une plage d'environ 1,8288 mm (0,072 pouce) à 1,9685 mm (0,0775 pouce) .

12. Système d'ablation par micro-ondes selon la revendication 1, dans lequel la plage de fréquence prédéterminée comprend 915 MHz et/ou 2,45 GHz et/ou 5,8 GHz.

ULTRASOUND WORKSTATION
150

131

130

140

174

172

180

170

160

110

100

120

121

FIG. 1

FIG. 2

EP 3 958 776 B1

FIG. 3A

FIG. 3B

FIG. 3C

FIG. 4

**FIG. 5A**

FIG. 5B

FIG. 5C

FIG. 6

EP 3 958 776 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62839008
- WO 2016054156 A1
- US 20160058507 A, Dickhans
- US 9247992 B, Ladtkow
- US 9119650 B, Brannan
- US 20160174873 A, Greenburg
- US 20160317224, Girotto
- US 20160000302, Brown